# EUROPÄISCHE PATENTSCHRIFT

(11) **EP 0 747 447 B1**
(45) Veröffentlichungstag und Bekanntmachung des Hinweises auf die Patenterteilung: **16.08.2001**
(21) Anmeldenummer: 96109101.4
(22) Anmeldetag: 06.06.1996
(51) Int. Cl.: C09B 19/00, G01N 33/533, C07D 498/14

(54) **Neue Oxazinfarbstoffe und ihre Verwendung als Fluoreszenzmarker**
Novel oxazine dyes and their use as fluorescent label
Nouveaux colorants oxaziniques et leur utilisation comme marquers fluorescents

(30) Priorität: 10.06.1995 DE 19521231
(43) Veröffentlichungstag der Anmeldung: 11.12.1996
(73) Patentinhaber: Roche Diagnostics GmbH, 68298 Mannheim (DE)
(72) Erfinder: Herrmann, Rupert, Dr., 82362 Weilheim (DE); Josel, Hans-Peter, Dr., 82362 Weilheim (DE); Drexhage, Karl-Heinz, Prof. Dr., 57076 Siegen (DE); Marx, Nicolaas-Joseph, 57399 Kirchhundem (DE)

(56) Entgegenhaltungen:
- EP-A- 0 543 333
- EP-A- 0 545 314
- EP-A- 0 608 737
- FR-A- 2 121 198
- US-A- 4 714 763
- US-A- 5 149 807

## Beschreibung

Die Erfindung betrifft neue kupplungsfähige Oxazinfarbstoffe sowie deren Verwendung als Fluoreszenzmarker in Konjugaten.

Zur Durchführung immunologischer Assays und in der DNA-Analytik werden Marker oder Label benötigt, die nach Ablauf einer analytspezifischen Reaktion eine Quantifizierung des Analyten erlauben.

Aufgrund der hohen Sensitivität haben sich in der letzten Zeit besonders fluorometrische Marker durchgesetzt. So ermöglicht die Markierung eines Antikörpers oder eine Nukleotids mit Fluoreszenzfarbstoffen eine direkte Quantifizierung.

Weit verbreitete Fluoreszenzfarbstoffe sind beispielsweise FITC (Fluoreszeinisothiocyanat), FLUOS (Fluoreszein N-Hydroxysuccinimidester), Resorufin und Rhodaminlabel, die aber für ihre Anregung relativ aufwendige Lichtquellen, zum Beispiel Argonlaser, benötigen.

Die rasche Entwicklung preiswerter Laserdioden mit einem Emissionsbereich von 630 - 780 nm, die sich zudem hervorragend zum Aufbau miniaturisierter Systeme eignen, macht Farbstoffe wünschenswert, die in diesen Wellenlängenbereichen absorbieren.

In EP-A-0 543 333 werden pentazyklische Rhodamin-Farbstoffe beschrieben, die als Label eingesetzt werden können. Die Schwerpunkte der Absorption liegen nur im Bereich bis 660 nm.

In der WO 88/047 77 werden Phthalocyaninfarbstoffe beschrieben, die allerdings mehr als eine funktionelle Gruppe besitzen, so daß sie bei Konjugation, zum Beispiel mit Antikörpern, zu Vernetzungen und Produktgemischen führen, die einen großen Reinigungsaufwand erfordern.

In US-P-5,149,807 sind pentazyklische Oxazinderivate als Laserfarbstoffe beschrieben. Diese verfügen aber über keine funktionelle Gruppe und sind somit nicht für eine spezifische Kupplung an biologische Moleküle wie Proteine, Haptene und Nukleinsäuren geeignet, noch wird eine solche Verwendung angesprochen.

Aus FR-A-2 121 198 sind Oxazinfarbstoffe bekannt, welche jedoch eine anders geartete Grundstruktur oder andere Substituenten aufweisen, als die mit der vorliegenden Erfindung entwickelten und beschriebenen Farbstoffe.

In US-A-4,714,763 sind Harnstoff-Derivate von Oxazin- oder Thiazin-Farbstoffen beschrieben. Oxazine, welche eine Verbrückung zwischen einer exozyklischen Aminogruppe und dem aus drei Ringen bestehenden Oxazingrundkörper beinhalten, sind dort nicht offenbart.

Aufgabe der vorliegenden Erfindung ist es, Farbstoffe zur Verfügung zu stellen, die sich für eine Kupplung mit biologischen Molekülen eignen, die über eine hohe Quantenausbeute verfügen, in einem Absorptionsbereich von 645 - 700 nm absorbieren und eine möglichst geringe unspezifische Bindung an biologische Verbindungen oder an Festphasen aufweisen.

Gelöst wird die Aufgabe durch die Erfindung, wie sie in den Ansprüchen charakterisiert ist.

Gegenstand der Erfindung sind funktionell kupplungsfähige Oxazinderivate der allgemeinen Formel I 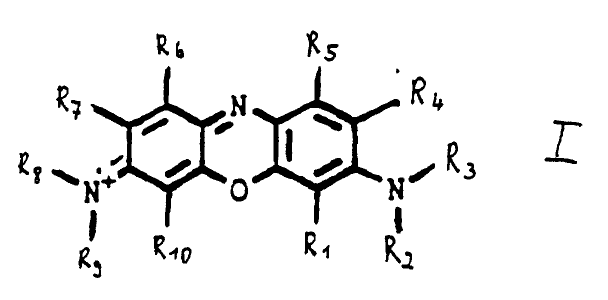
worin R₁, R₄, R₅, R₆, R₇, R₁₀
   Wasserstoff, Alkyl, Alkoxy, Hydroxy, Halogen, Carboxyl, Sulfonyl oder Amino darstellt
   und
R₂, R₃
   Wasserstoff, Alkyl, Alkoxy, Polyoxyhydrocarbyleinheiten, Phenyl, Phenylalkyl bedeuten, die durch Hydroxy, Halogen, Sulfonyl, Carboxy, Amino, Alkoxycarbonyl substituiert sein können, wobei R2 mit R1 oder R3 mit R4 eine gesättigte oder ungesättigte C2- oder C3-Brücke bilden kann oder R2 mit R3 eine gesättigte oder ungesättigte C4- oder C5-Brücke bilden kann und
R8, R9
   Wasserstoff, Alkyl, Alkoxy, Polyoxyhydrocarbyleinheiten, Phenyl, Phenylalkyl bedeutet, die durch Hydroxy, Halogen, Sulfonyl, Carboxy, Amino, Alkoxycarbonyl substituiert sein kann, wobei R8 mit R7 oder R9 mit R10 eine gesättigte oder ungesättigte C2- oder C3-Brücke oder R8 mit R9 eine gesättigte oder ungesättigte C4- oder C5-Brücke bilden kann
und wobei mindestens einer der Reste R2, R3, R8 oder R9 einen nicht-brückebildenden Rest darstellt, der mit einer kupplungsfähigen aktivierten oder zu einer Kupplung aktivierbaren Gruppe substituiert ist
und wobei mindestens einer der Reste R2, R3, R8 oder R9 einen brückebildenden Rest, der gegebenenfalls durch Alkyl, substituiert sein kann, darstellt.

Bevorzugt bildet R3 mit R4 und / oder R7 mit R8 eine gesättigte oder ungesättigte C3-Brücke.

Ganz besonders bevorzugt bilden R3 mit R4 und / oder R7 mit R8 eine C3-Brücke, während R2 und / oder R9 nichtbrückebildende Substituenten, bevorzugt Alkyl darstellen, wobei mindestens ein nicht-brückebildender Substituent mit einer kupplungsfähig aktivierten oder aktivierbaren Gruppe substituiert ist.

Unter dem Begriff "Polyoxyhydrocarbyl-Einheiten" im Sinne der vorliegenden Erfindung sind polymere oder oligomere organische Reste zu verstehen, die über O-Brücken miteinander verknüpft sind. Insbesondere sind unter diesem Begriff Polyether, Polyole, lösliche Carbohydrate, Derivate davon oder wasserlösliche Polymere zu verstehen. Besonders bevorzugt sind Polyethylenoxygruppen, deren Größe so ist, daß das Molekulargewicht der Gesamtverbindung 800 - 1200, vorzugsweise etwa 1000 ist. Die oben genannten Polyethylenoxygruppen bewirken eine Verbesserung der Löslichkeit, vermindern die unspezifische Bindung der Verbindungen an Proteine und verhindern eine Dimerisierung.

Eine Alkylgruppe hat 1-10, bevorzugt 1-7 Kohlenstoffatome und kann verzweigtkettig oder geradkettig sein; sie besitzt ganz besonders bevorzugt 1-4 Kohlenstoffatome und ist insbesondere zum Beispiel Methyl, Ethyl, Propyl, iso-Propyl, Butyl, iso-Butyl oder tert.-Butyl.

Eine Phenylalkylgruppe mit bevorzugt 1-3 Kohlenstoffatomen in der Alkylgruppe ist insbesondere eine Phenethyl- oder Benzylgruppe.

Unter Halogen wird Fluor, Chlor, Brom oder Jod, bevorzugt Chlor verstanden.

Eine Alkoxygruppe in einer Alkoxycarbonylgruppe hat 1-10, bevorzugt 1-4, ganz besonders bevorzugt 1 oder 2 C-Atome.

In den erfindungsgemäßen Verbindungen der Formel I liegt vorzugsweise zumindest einer der Reste R2, R3, R8, R9 als ein nicht-brückenbildender Rest vor, der mit einer kupplungsfähigen aktivierten oder zu einer Kupplung aktivierbaren Gruppe substituiert ist. Eine solche aktivierte Gruppe leitet sich insbesondere von einer aktivierbaren Carbonsäure- oder Sulfonsäuregruppierung ab, und ist zum Beispiel ein Säureester, ein Säureanhydrid, ein Säurehalogenid, vorzugsweise Bromid, insbesondere Chlorid oder ein N-Hydroxy-Succinimidester. Zwischen aktivierter Gruppe und dem nicht-brückenbildendem Rest kann noch eine Linkerverbindung, beispielsweise DADOO zwischengeschaltet sein.

Die Tabelle gibt einige Beispiele für aktivierte, kupplungsfähige Gruppen. Dem Fachmann sind weitere solcher Gruppen aus der Synthesechemie für Konjugate bekannt.

**Tabelle 1**

| **Aktivierte Gruppe** | **Verknüpfung mit** | **Produkt** |
|---|---|---|
| NHS-Ester | Amine | Amid |
| Isothiocyanat | Amine | Thioharnstoff |
| Gemischtes Anhydrid | Amine | Amid |
| Maleimid | Thiol | Thioether |
| Thiol | Maleimid | Thioether |
| Haloacetyl | Thiol | Thioether |
| Hydrazine | Aldehyd | Hydrazone |
| Amine | Aldehyd | Amin (n. Reduktion) |
| Amine | reaktive Carbonsäure | Amide |

Als Gegenion läßt sich jedes zur Ladungsneutralisierung geeignete und mit dem kationischen Grundgerüst kompatible Anion verwenden; bevorzugt wird Perchlorat eingesetzt, oder aber das Gegenion von einer Carboxy- oder Sulfongruppe einer der Reste abgeleitet. Durch Wahl eines geeigneten Gegenions läßt sich zusätzlich zur Auswahl und Kombination der Reste der je nach dem beabsichtigten Anwendungszweck gewünschte Grad der Lipophilie optimieren.

Beispiele für besonders bevorzugte Substituenten in der Bedeutung von R₂, R₃, R₈, oder R₉ sind:
Wasserstoff, Methyl, Carboxymethyl, Ethyl, Carboxyethyl, 3-Sulfopropyl, 4-Sulfobutyl, 3-Carboxypropyl, 4-Carboxybutyl, 3-Methoxycarbonylpropyl, 3-Ethoxycarbonylpropyl, Methoxy-ethoxy-ethyl, Hydroxy-ethoxy-ethyl, Benzyl.

Zur Verwendung als hydrophile Marker kann es zweckmäßig sein, unsymmetrisch substituierte Produkte einzusetzen, in denen die Reste R₂, R₃ gegenüber R₈, R₉ verschieden sind, und zum Beispiel eine 3-Carboxypropyl oder 4-Carboxybutylgruppe (R₂ oder / und R₃) und eine 3-Sulfo-Propyl- oder 4-Sulfo-Butyl-Gruppe (R₈ und / oder R₉) bedeuten.

Besonders bevorzugte Reste in Verbindungen der Formel I sind:
R₁, R₄, R₅, R₆ und / oder R₁₀ = Wasserstoff
R₃ mit R₄ und / oder R₈ mit R₇ = (CH₂)₃, 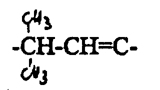
R₁ mit R₂ oder R₉ mit R₁₀ = (CH₂)₃
R₂, R₃, R₈, R₉ = -CH₂-CH₃, -CH₂-CH₂-CH₂-COOH
R₂ mit R₃ oder R₈ mit R₉ = (CH₂)₄

Ein weiterer Gegenstand der Erfindung sind mit den erfindungsgemäßen Fluoreszenzfarbstoffen gekuppelte biologisch aktive Substanzen (Konjugate) der Formel II 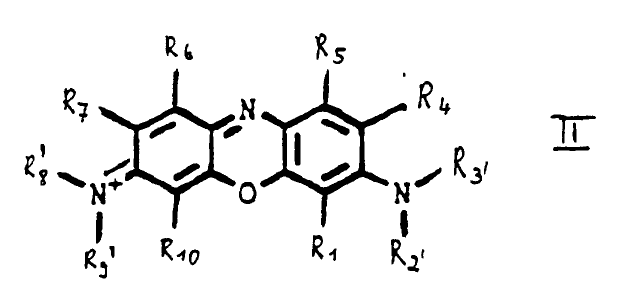 worin R₁, R₄, R₅, R₆, R₇, R₁₀ die oben angegebene Bedeutung haben.
R2', R3',
   Wasserstoff, Alkyl, Alkoxy, Polyoxyhydroxycarbonyleinheiten, Phenyl, Phenylalkyl bedeutet, die durch Hydroxy, Sulfonyl, Carboxy, Amino, Alkoxycarbonyl substituiert sein können, wobei R2' mit R₁ oder R3' mit R4 eine gesättigte oder ungesättigte C4- oder C5-Brücke bilden kann und
R8', R9',
   Wasserstoff, Alkyl, Alkoxy, Polyoxyhydroxycarbonyleinheiten, Phenyl, Phenylalkyl bedeutet, die durch Hydroxy, Sulfonyl, Carboxy, Amino, Alkoxycarbonyl substituiert sein können, wobei R2' mit R₁ oder R3' mit R4 eine gesättigte oder ungesättigte C4- oder C5-Brücke bilden kann
   und
wobei mindestens einer der Reste R2', R3', R8' oder R9' einen nicht-brückebildenden Rest darstellt, der mit einer biologisch aktiven Substanz gekuppelt ist und wobei mindestens einer der Reste R2', R3', R8' und R9' einen nicht-brückebildenden Rest darstellt, der gegebenenfalls durch Alkyl substituiert ist.

Unter einer biologisch aktiven Substanz wird insbesondere ein Hapten, Antigen, Antikörper oder Fragment davon, Protein oder Mono- oder Polynukleotid (PNA, RNA oder DNA-Molekül) verstanden.

Die Verbindungen der Formel I lassen sich durch Kondensation von 1,3-Aminophenolen der Formel III mit Nitrosoaminophenolen der Formel IV erhalten.

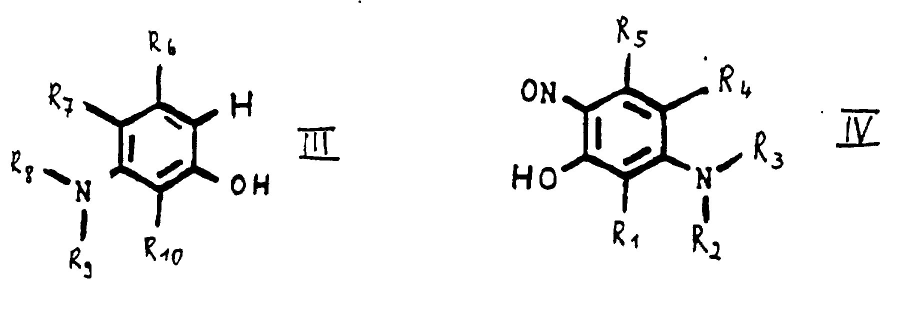

Aktivierbare Gruppen werden nach bekannten Methoden zu kupplungsfähigen Gruppen aktiviert und mit reaktiven Gruppen biologisch aktiver Moleküle zu Konjugaten der Formel II gekuppelt. Dabei können auch zwischen den aktivierten Gruppen und den biologisch aktiven Molekülen noch Linker eingebaut werden.

Mit den erfindungsgemäßen Verbindungen werden neue Verbindungen bereitgestellt, die sich aufgrund ihrer spektroskopischen Eigenschaften (Absorptionsmaximum im Bereich zwischen 645 bis 700 nm) sehr gut für kupplungsfähige Absorptionsfarbstoffe, insbesondere Fluoreszenzfarbstoffe für die Anwendung in Hapten- / und Antikörperprotein-Konjugaten, zur Polynukleotidmarkierung und zur Anfärbung von Latices (Fluoreszenzlatices) eignen. Die Quantenausbeute ist hoch und liegt zwischen 40 und 70% in ethanolischer Lösung.

Für die Anwendung in Hapten- / Antikörper- /Protein- oder Polynukleotidkonjugaten ist es vorteilhaft, wenn die Farbstoffe gut wasserlöslich sind. Für diesen Verwendungszweck werden deshalb vorzugsweise Verbindungen der allgemeinen Formel I eingesetzt, in denen R₂, R₃, R₈, R₉ möglichst hydrophil sind. Vorzugsweise sind diese Verbindungen unsymmetrisch substituierte Produkte, die zum Beispiel sowohl Carboxyl- als auch Sulfonsäuregruppen enthalten. Die Kupplung zum Konjugat erfolgt über mindestens einen der aktivierten Substituenten der Reste R₂, R₃, R₈ oder R₉ und insbesondere über eine Hydroxysuccinimidgruppierung.

Konjugate der Fluoreszenzfarbstoffe mit Haptenen, wie zum Beispiel Theophylin, Digoxin, T3, T4 oder Protein, wie zum Beispiel Antikörper, eignen sich zum Beispiel zum Einsatz in diagnostischen Systemen, insbesondere für Fluoreszenzimmunoassays.

Weiterer Gegenstand der vorliegenden Erfindung ist ein Verfahren zur Bestimmung einer ersten immunologischen bindefähigen Substanz, das dadurch gekennzeichnet ist, daß ein Konjugat einer erfindungsgemäßen Verbindung mit einer zweiten immunologisch bindefähigen Substanz, die gleich oder verschieden mit der ersten Substanz sein kann, verwendet wird und daß die durch eine immunologische Bindungsreaktion, welche für die erste Substanz spezifisch ist, verursachte Absorptions- oder Fluoreszenzänderung oder Fluoreszenzpolarisationsänderung der erfindungsgemäßen Verbindung als Maß für die Menge der in der Probe enthaltenden zu bestimmenden Substanz bestimmt wird.

Ein weiterer Gegenstand der Erfindung ist die Verwendung der erfindungsgemäßen Konjugate für Immunoassays.

Die erfindungsgemäßen kupplungsfähigen Verbindungen der Formel I eignen sich auch zur Herstellung von Konjugaten mit Mono- oder Polynukleotiden oder PNA.

Ein weiterer Gegenstand der Erfindung ist daher die Verwendung dieser Konjugate zur DNA-Analytik.

### Beispiel 1

### Synthese von I

0,6 g (2,3 mmol) γ-(7-Hydroxy-1,2,3,4-tetrahydrochinol-1-yl)-buttersäureethylester und 0,5 g (2,4 mmol) N-Ethyl-7-hydroxy-6-nitroso-1,2,3,4-tetrahydrochinolin werden in 12 ml Ethanol nach Zusatz von 2 ml 2,5 m Salzsäure 2 Stunden unter Rückfluß gekocht. Die Lösung wird am Rotationsverdampfer bis zur Trocknung eingedampft. Der Rückstand wird in Ethanol aufgenommen und über Aluminiumoxid chromatographisch vorgereinigt. Der so erhaltene Ethylester des Zielfarbstoffes hat das Absorptionsmaximum in Ethanol bei 653 nm.

Der Ethylester wird in einem Gemisch aus 30 ml Aceton, 20 ml Wasser und 1 ml 2,5 m Salzsäure 30 min. unter Rückfluß gekocht. Zur Reinigung wird der Farbstoff über Silicagel chromatographiert (Laufmittel: zunächst Aceton-Chloroform 3:1, dann Aceton und schließlich Ethanol). Man erhält 0,5 g I

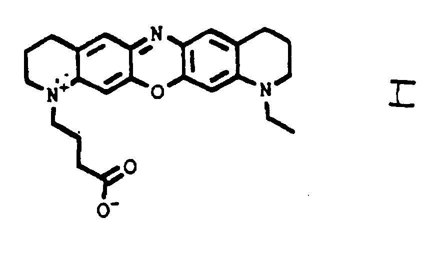

### Beispiel 2

### Oxazin-N-Hydroxysuccinimidester II

40 mg Oxazin I werden mit 10 mg N-Hydroxysuccinimideesteer und 19 mg Dicyclohexylcarbodiimid in 20 ml Acetonitril gelöst. Man läßt 4 h bei Raumtemperatur rühren und rotiert das Produktgemisch ein. Die Reinigung erfolgt über Reverse Phase-Kieselgel.

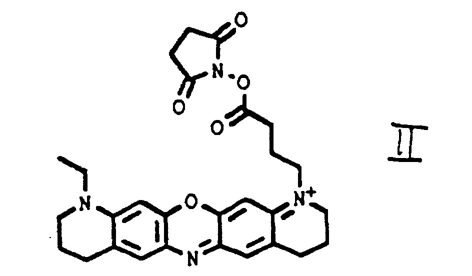

Digoxin-3-Carboxymethylether-Diaminodioxooctan-Konjugat III (Dig-CME-DADOO)

Das Hapten-Fluoreszenz-Konjugat wird durch Umsetzung von 11 mg I und 17,5 mg Dig-DADOO 18 h bei Raumtemperatur in Acetonitril erhalten. Der Ansatz wird einrotiert und anschließend über Kieselgel, Eluens Chloroform-Methanol-Essigsäure 3:1:0:1 aufgereinigt.

Ausbeute: 4 mg

Analytik: MS entspricht

### Labeling von Proteinen mit II

10 mg Protein, z.B. MAK <TSH> werden in I ml Natriumhydrogenphosphat-Puffer pH 8 gelöst. Dazu gibt man eine Lösung eines 10-fachen molaren Überschusses an II, gelöst in 500 ul DMSO. Die Reaktionslösung wird 1 h bei Raumtemperatur geschüttelt. Das Konjugat wird über eine Sephadex G 50 Säule, Laufmittel = Puffer, gereinigt, dreimal gegen Wasser dialysiert und lyophilisiert.

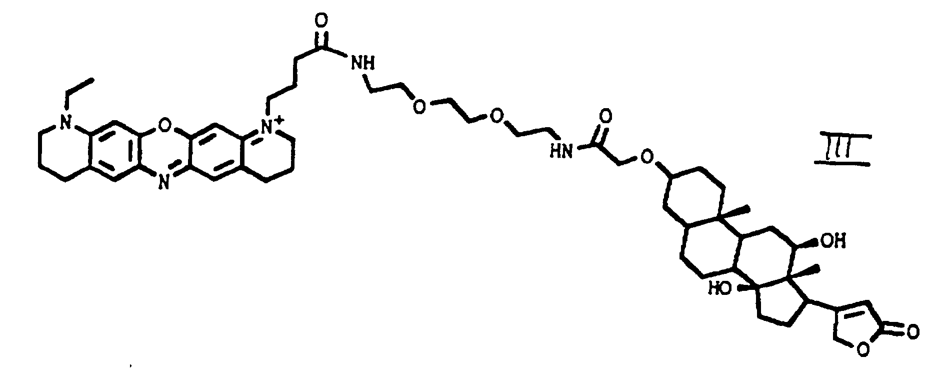

### Beispiel 3

### Synthese von Oxazinen der Formeln IV-X mit aktivierbaren COOH-Gruppen

3 mmol substituiertes m-Aminophenol bzw. m-Aminoanisol und 33 mmol 6-Nitroso-3-aminophenol werden in einem Gemisch von 20 ml Äthanol und 1 ml 2.5 N Salzsäure gelöst und zum Rückfluß erhitzt. Die dabei stattfindende Farbstoffbildung wird spektrometrisch verfolgt (λmax im Bereich 650 - 700 nm). Die Reaktion wird abgebrochen, wenn die Farbstoffkonzentration nicht mehr zunimmt.

Die so erhaltene Lösung wird auf Volumen von ca. 10 ml eingeengt und dann tropfenweise zu 200 ml 10%iger wäßriger NaBF₄-Lösung hinzugefügt. Das Farbstoff-tetrafluoroborat fällt hierbei vollständig aus. Nach Abdekantieren der überstehenden Flüssigkeit und Filtrieren wird der Rückstand in 100 ml Dichlormethan aufgenommen und diese Lösung 3 mal mit je 100 ml Wasser gewaschen. Die Lösung wird über Na₂SO₄ getrocknet und einrotiert. Der Farbstoff fällt hierbei als zähes, fast schwarzes Öl an. Ausbeute: 40-60%.

### Hydrolyse

Der Rohfarbstoff(Ethylester) wird in einem Gemisch von 30 ml Aceton, 15 ml Wasser und 1 ml 2.5 N Salzsäure gelöst und zum Rückfluß erhitzt. Die Hydrolyse wird dünnschicht-chromatographisch verfolgt (Silicagel, MeOH/H₂O 3:1). Nach praktisch vollständiger Umsetzung wird die Reaktionslösung bei ca. 30°C einrotiert und der Rückstand chromatographisch gereinigt.

| | Reaktionsdauer | Hydrolysedauer | Reinigung | λ. max. EtOH |
|---|---|---|---|---|
| IV | 1,5 h | 7 h | -- | 660 |
| V | 70 min. | 9 h | Silicagel LM=Chloroform/EtOH | 650 |
| VI | 75 min. | 9h | Silicagel LM=Chloroform/EtOH | 649 |
| VII | 40 min. | 10 h | Silicagel LM=Chloroform/EtOH | 672 |
| VIII | 90 min. | 48 h | Silicagel LM=Chloroform/EtOH | 682 |
| IX | 3 h | 24 h | Silicagel LM=Chloroform/EtOH | 673 |
| X | 60 min. | 24 h | Silicagel LM=Chloroform/EtOH | 672 |

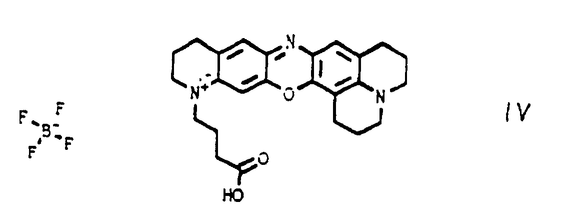

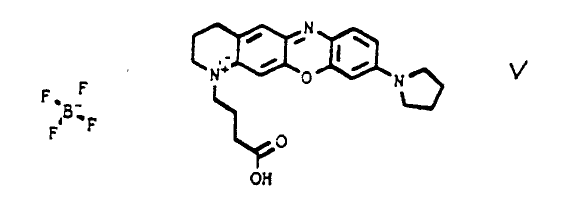

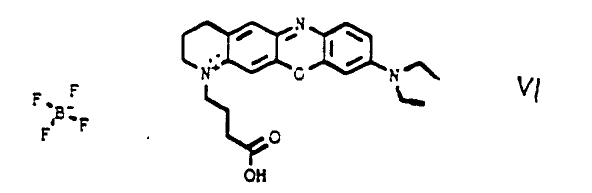

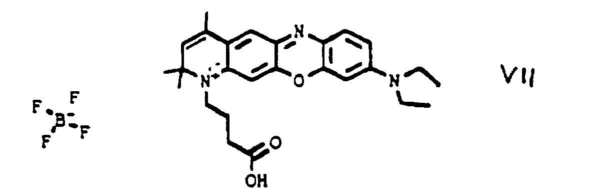

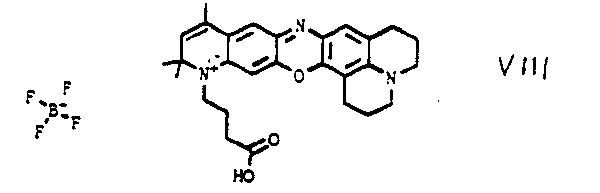

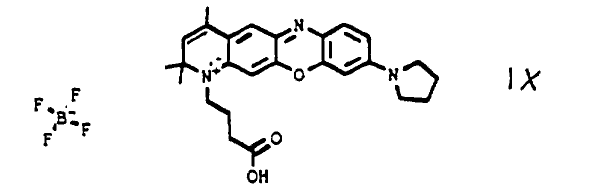

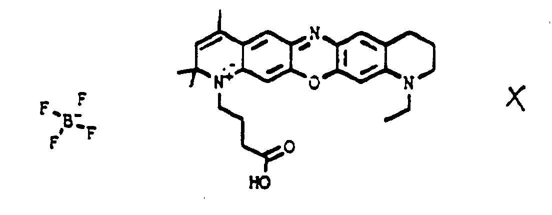

## Patentansprüche

1. Oxazinderivate der allgemeinen Formel I 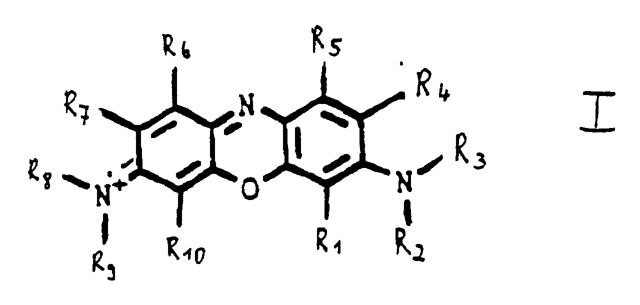
worin R₁,R₄,R₅,R₆,R₇ und R₁₀ Wasserstoff, Alkyl, Hydroxy, Halogen, Carboxyl, Sulfonyl oder Amino bedeutet und
R₂, R₃,
Wasserstoff, Alkyl, Alkoxy, Polyoxyhydrocarbyleinheiten, Phenyl, Phenylalkyl bedeuten, die durch Hydroxy, Sulfonyl, Carboxy, Amino, Alkoxycarbonyl substituiert sein können, wobei R₂ mit R₁ oder R₃ mit R₄ eine gesättigte oder ungesättigte C2- oder C3-Brücke bilden kann oder R₂ mit R₃ eine gesättigte oder ungesättigte C4- oder C5-Brücke bilden kann und
R₈, R₉
Wasserstoff, Alkyl, Alkoxy, Polyoxyhydrocarbyleinheiten, Phenyl, Phenylalkyl bedeuten, die durch Hydroxy, Sulfonyl, Carboxy, Amino, Alkoxycarbonyl substituiert sein können, wobei R₈ mit R₇ oder R₉ mit R₁₀ eine gesättigte oder ungesättigte C2- oder C3-Brücke oder R₈ mit R₉ eine gesättigte oder ungesättigte C4- oder C5-Brücke bilden kann
und wobei mindestens einer der Reste R₂, R₃, R₈ oder R₉ einen nicht-brückebildenden Rest darstellt, der zusätzlich mit einer kupplungsfähigen aktivierten oder zu einer Kupplung aktivierbaren Gruppe substituiert ist
und wobei mindestens einer der Reste R₂, R₃, R₈ oder R₉ einen brückebildenden Rest, der gegebenenfalls durch Alkyl substituiert sein kann, darstellt.

2. Oxazinderivate gemäß Anspruch 1, dadurch gekennzeichnet, daß R₃ mit R₄ und / oder R₇ mit R₈ eine gesättigte oder ungesättigte C3-Brücke bildet.

3. Oxazinderivat gemäß Anspruch 1 oder 2, dadurch gekennzeichnet, daß R₂ und / oder R₉ nicht-brückebildende Substituenten darstellen, von denen mindestens einer zusätzlich mit einer kupplungsfähigen aktivierten oder zu einer Kupplung aktivierbaren Gruppe substituiert ist.

4. Oxazinderivat gemäß Anspruch 3, dadurch gekennzeichnet, daß ein nicht-brückebildender Substituent einen mit einer aktivierbaren oder aktivierten Gruppe substituierter Alkylrest darstellt.

5. Oxazinderivate gemäß Anspruch 3 oder 4, dadurch gekennzeichnet, daß die aktivierbare Gruppe eine Carbonsäure oder Sulfonsäuregruppe darstellt oder die aktivierte Gruppe ein Säurester, Säureanhydrid, Säurehalogenid oder N-Hydroxysuccinimidester darstellt.

6. Oxazinfarbstoffkonjugate der Formel II 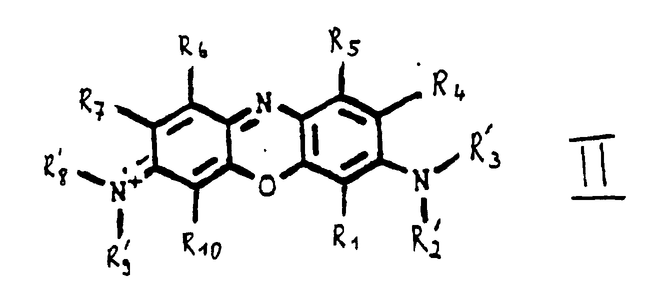
worin R₁,R₄,R₅,R₆,R₇ und R₁₀ Wasserstoff, Alkyl, Hydroxy, Halogen, Carboxyl, Sulfonyl oder Amino bedeutet und
R2',R3',
Wasserstoff, Alkyl, Alkoxy, Polyoxyhydrocarbyleinheiten, Phenyl, Phenylalkyl bedeuten, die durch Hydroxy, Sulfonyl, Carboxy, Amino, Alkoxycarbonyl substituiert sein können, wobei R2' mit R₁ oder R3' mit R₄ eine gesättigte oder ungesättigte C2- oder C3-Brücke bilden kann oder R₂' mit R₃' eine gesättigte oder ungesättigte C4- oder C5-Brücke bilden kann und
R8', R9'
Wasserstoff, Alkyl, Alkoxy, Polyoxihydrocarbyleinheiten, Phenyl, Phenylalkyl bedeutet, die durch Hydroxy, Sulfonyl, Carboxy, Amino, Alkoxycarbonyl substituiert sein können, wobei R8' mit R₇ oder R9' mit R₁₀ eine gesättigte oder ungesättigte C3-Brücke oder R8' mit R9' eine gesättigte oder ungesättigte C4- oder C5-Brücke bilden kann
und wobei mindestens einer der Reste R2', R3', R8' oder R9' einen nicht-brückebildenden Rest darstellt, der zusätzlich mit einer biologisch aktiven Gruppe substituiert ist
und wobei mindestens einer der Reste R2', R3', R8' oder R9' einen brückebildenden Rest, der gegebenenfalls durch Alkyl substituiert sein kann, darstellt.

7. Oxazinkonjugat gemäß Anspruch 6, dadurch gekennzeichnet, daß R3' mit R4 und / oder R7 mit R8' eine gesättigte oder ungesättigte C3-Brücke bildet.

8. Oxazinkonjugat gemäß Anspruch 6 oder 7, dadurch gekennzeichnet, daß R2' und / oder R9' nicht-brückebildende Substituenten darstellen, wobei mindestens ein Substituent mit einer biologisch aktiven Gruppe substituiert ist.

9. Oxazinkonjugat gemäß einem der Ansprüche 6 bis 8, dadurch gekennzeichnet, daß die biologisch aktive Gruppe ein Hapten, Antigen, Antikörper oder Protein darstellt.

10. Oxazinkonjugat gemäß einem der Ansprüche 6 bis 8, dadurch gekennzeichnet, daß die biologisch aktive Gruppe ein Mono- oder Polynukleotid darstellt.

11. Verfahren zur Bestimmung einer ersten immunologisch bindefähigen Substanz, dadurch gekennzeichnet, daß ein Konjugat gemäß Anspruch 9 mit einer zweiten immunologisch bindefähigen Substanz,, die gleich oder verschieden mit der ersten Substanz sein kann, verwendet wird und daß die durch eine immunologische Bindungsreaktion, welche für die erste Substanz spezifisch ist, verursachte Absorption oder Fluoreszenzänderung oder Fluoreszenzpolarisationsänderung als Maß für die Menge der in der Probe enthaltenden zu bestimmenden Substanz bestimmt wird.

12. Verwendung eines Konjugates gemäß Anspruch 9 für Immunoassays.

13. Verwendung eines Konjugates gemäß Anspruch 10 zur DNA-Analytik.

14. Verfahren zur Herstellung der Oxazinderivate der Formel I gemäß Anspruch 1, dadurch gekennzeichnet, daß 1,3 Aminophenole der Formel III mit Nitrosoaminophenolen der Formel IV kondensiert werden.

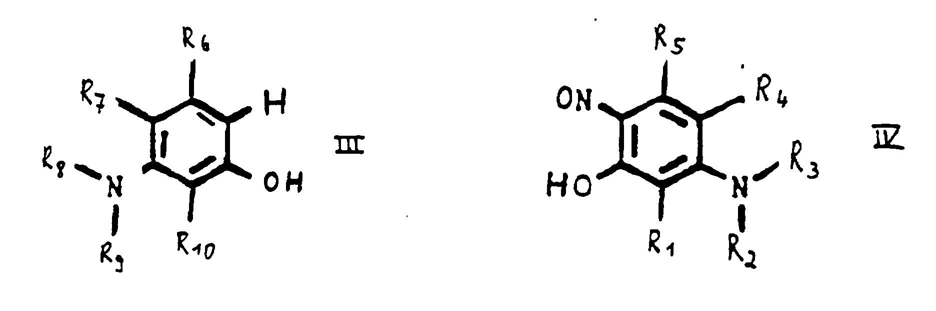

## Claims

1. Oxazine derivatives of the general formula I 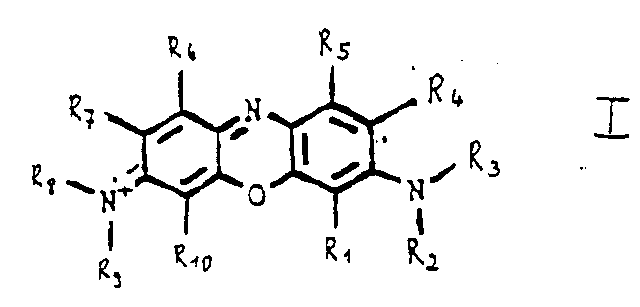
wherein R₁, R₄, R₅, R₆, R₇ and R₁₀ denote hydrogen, alkyl, hydroxy, halogen, carboxyl, sulfonyl or amino and
R₂, R₃
denote hydrogen, alkyl, alkoxy, polyoxyhydrocarbyl units, phenyl, phenylalkyl which can be substituted by hydroxy, sulfonyl, carboxy, amino, alkoxycarbonyl, and R₂ together with R₁ or R₃ together with R₄ can form a saturated or unsaturated C2 or C3 bridge or R₂ together with R₃ can form a saturated or unsaturated C4 or C5 bridge and
R₈, R₉
denote hydrogen, alkyl, alkoxy, polyoxyhydrocarbyl units, phenyl, phenylalkyl which can be substituted by hydroxy, sulfonyl, carboxy, amino, alkoxycarbonyl, and R₈ together with R₇ or R₉ together with R₁₀ can form a saturated or unsaturated C2 or C3 bridge or R₈ together with R₉ can form a saturated or unsaturated C4 or C5 bridge
and wherein at least one of the residues R₂, R₃, R₈ or R₉ is a non-bridging residue which is additionally substituted with an activated group with coupling capability or with a group that can be activated to couple
and wherein at least one of the residues R₂, R₃, R₈ or R₉ is a bridging residue which can optionally be substituted by alkyl.

2. Oxazine derivatives as claimed in claim 1, wherein R₃ together with R₄ and/or R₇ together with R₈ form a saturated or unsaturated C3 bridge.

3. Oxazine derivative as claimed in claim 1 or 2, wherein R₂ and/or R₉ are non-bridging substituents of which at least one is additionally substituted with an activated group with coupling capability or with a group that can be activated to couple.

4. Oxazine derivative as claimed in claim 3, wherein a non-bridging substituent represents an alkyl residue substituted with a group that can be activated or with an activated group.

5. Oxazine derivatives as claimed in claim 3 or 4, wherein the group that can be activated is a carboxylic acid or sulfonic acid group or the activated group is an acid ester, acid anhydride, acid halogenide or N-hydroxysuccinimide ester.

6. Oxazine dye conjugates of formula II 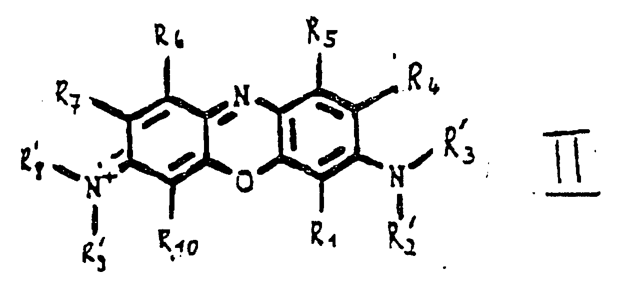
wherein R₁, R₄, R₅, R₆, R₇ and R₁₀ denote hydrogen, alkyl, hydroxy, halogen, carboxyl, sulfonyl or amino and
R₂', R₃'
denote hydrogen, alkyl, alkoxy, polyoxyhydrocarbyl units, phenyl, phenylalkyl which can be substituted by hydroxy, sulfonyl, carboxy, amino, alkoxycarbonyl, and R₂' together with R₁ or R₃' together with R₄ can form a saturated or unsaturated C2 or C3 bridge or R₂' together with R₃' can form a saturated or unsaturated C4 or C5 bridge and
R₈' R₉'
denote hydrogen, alkyl, alkoxy, polyoxyhydrocarbyl units, phenyl, phenylalkyl which can be substituted by hydroxy, sulfonyl, carboxy, amino, alkoxycarbonyl, and R₈' together with R₇ or R₉' together with R₁₀ can form a saturated or unsaturated C3 bridge or R₈' together with R₉' can form a saturated or unsaturated C4 or C5 bridge
and wherein at least one of the residues R₂', R₃', R₈' or R₉' is a non-bridging residue which is additionally substituted with a biologically active group
and wherein at least one of the residues R₂', R₃', R₈' or R₉' is a bridging residue which can optionally be substituted by alkyl.

7. Oxazine conjugate as claimed in claim 6, wherein R₃' together with R₄ and/or R₇ together with R₈' form a saturated or unsaturated C3 bridge.

8. Oxazine conjugate as claimed in claim 6 or 7, wherein R₂' and/or R₉' are non-bridging substituents of which at least one substituent is substituted with a biologically active group.

9. Oxazine conjugate as claimed in one of the claims 6 to 8, wherein the biologically active group is a hapten, antigen, antibody or protein.

10. Oxazine conjugate as claimed in one of the claims 6 to 8, wherein the biologically active group is a mononucleotide or polynucleotide.

11. Method for determining a first immunologically bindable substance, wherein a conjugate as claimed in claim 9 is used together with a second immunologically bindable substance which can be the same as or different from the first substance and wherein the absorption or fluorescence change or fluorescence polarization change caused by an immunological binding reaction which is specific for the first substance is determined as a measure for the amount of the substance to be determined in the sample.

12. Use of a conjugate as claimed in claim 9 for immunoassays.

13. Use of a conjugate as claimed in claim 10 for DNA analysis.

14. Process for the production of the oxazine derivatives of formula I as claimed in claim 1, wherein 1,3-aminophenols of formula III are condensed with nitrosoamino phenols of formula IV.

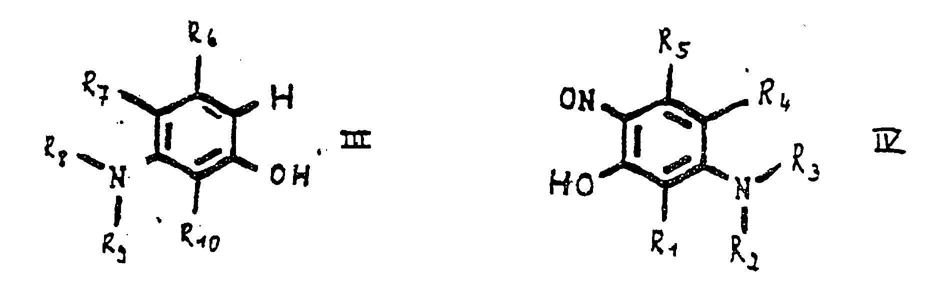

## Revendications

1. Dérivés oxaziniques répondant à la Formule générale I 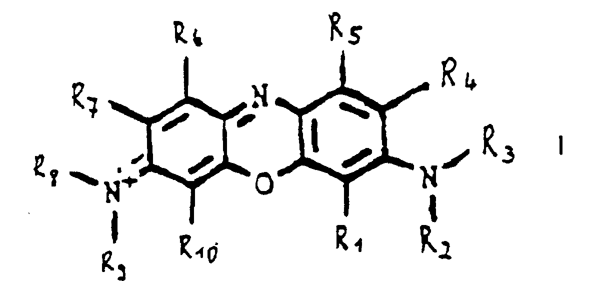
dans laquelle R₁, R₄, R₅, R₆, R₇ et R₁₀ signifient un atome d'hydrogène, un groupe alkyle, un groupe hydroxyle, un atome d'halogène, un groupe carboxyle, un groupe sulfonyle ou un groupe amino et
R₂, R₃,
signifient un atome d'hydrogène, un groupe alkyle, un groupe alcoxy, des unités polyoxyhydrocarbyles, un groupe phényle, un groupe phénylalkyle, qui peuvent être substitués par un groupe hydroxyle, un groupe sulfonyle, un groupe carboxyle, un groupe amino, un groupe alcoxycarbonyle, où R₂ avec R₁ ou R₃ avec R₄ peut former un pont en C₂ ou C₃ saturé ou insaturé ou R₂ avec R₃ peut former un pont en C₄ ou C₅ saturé ou insaturé et
R₈, R₉,
signifient un atome d'hydrogène, un groupe alkyle, un groupe alcoxy, des unités polyoxyhydrocarbyles, un groupe phényle, un groupe phénylalkyle, qui peuvent être substitués par un groupe hydroxyle, un groupe sulfonyle, un groupe carboxyle, un groupe amino, un groupe alcoxycarbonyle, où R₈ avec R₇ ou R₉ avec R₁₀ peut former un pont en C₂ ou C₃ saturé ou insaturé ou R₈ avec R₉ peut former un pont en C₄ ou C₅ saturé ou insaturé
et au moins un des résidus R₂, R₃, R₈ ou R₉ représentant un résidu ne formant pas de pont, qui est en outre substitué par un groupe activé apte au couplage ou activable au couplage
et au moins un des résidus R₂, R₃, R₈ ou R₉ représentant un résidu formant un pont, qui peut éventuellement être substitué par un groupe alkyle.

2. Dérivés oxaziniques selon la revendication 1, caractérisés en ce que R₃ avec R₄ et/ou R₇ avec R₈ forme un pont en C₃ saturé ou insaturé.

3. Dérivé oxazinique selon la revendication 1 ou 2, caractérisé en ce que R₂ et/ou R₉ représentent des substituants ne formant pas de pont, parmi lesquels au moins un est substitué en outre par un groupe activé apte au couplage ou activable au couplage.

4. Dérivé oxazinique selon la revendication 3, caractérisé en ce qu'un substituant ne formant pas de pont représente un résidu alkyle substitué par un groupe activable ou activé.

5. Dérivés oxaziniques selon la revendication 3 ou 4, caractérisés en ce que le groupe activable représente un groupe d'acide carboxylique ou d'acide sulfonique ou en ce que le groupe activé représente un ester d'acide, un anhydride d'acide, un halogénure d'acide ou un ester N-hydroxysuccinimidique.

6. Conjugués de colorants oxaziniques répondant à la Formule II 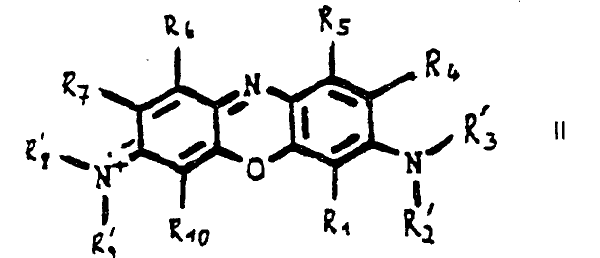
dans laquelle R₁, R₄, R₅, R₆, R₇ et R₁₀ signifient un atome d'hydrogène, un groupe alkyle, un groupe hydroxyle, un atome d'halogène, un groupe carboxyle, un groupe sulfonyle ou un groupe amino et
R₂', R₃',
signifient un atome d'hydrogène, un groupe alkyle, un groupe alcoxy, des unités polyoxyhydrocarbyles, un groupe phényle, un groupe phénylalkyle, qui peuvent être substitués par un groupe hydroxyle, un groupe sulfonyle, un groupe carboxyle, un groupe amino, un groupe alcoxycarbonyle, où R₂' avec R₁ ou R₃' avec R₄ peut former un pont en C₂ ou C₃ saturé ou insaturé ou R₂' avec R₃' peut former un pont en C₄ ou C₅ saturé ou insaturé et
R₈', R₉',
signifient un atome d'hydrogène, un groupe alkyle, un groupe alcoxy, des unités polyoxyhydrocarbyles, un groupe phényle, un groupe phénylalkyle, qui peuvent être substitués par un groupe hydroxyle, un groupe sulfonyle, un groupe carboxyle, un groupe amino, un groupe alcoxycarbonyle, où R₈' avec R₇ ou R₉' avec R₁₀ peut former un pont en C₃ saturé ou insaturé ou R₈' avec R₉' peut former un pont en C₄ ou C₅ saturé ou insaturé
et au moins un des résidus R₂', R₃', R₈' ou R₉' représentant un résidu ne formant pas de pont, qui est en outre substitué par un groupe biologiquement actif
et au moins un des résidus R₂', R₃', R₈' ou R₉' représentant un résidu formant un pont, qui peut éventuellement être substitué par un groupe alkyle.

7. Conjugué oxazinique selon la revendication 6, caractérisé en ce que R₃' avec R₄ et/ou R₇ avec R₈' forme un pont en C₃ saturé ou insaturé.

8. Conjugué oxazinique selon la revendication 6 ou 7, caractérisé en ce que R₂' et/ou R₉' représentent des substituants ne formant pas de pont, parmi lesquels au moins un est substitué par un groupe biologiquement actif.

9. Conjugué oxazinique selon l'une des revendications 6 à 8, caractérisé en ce que le groupe biologiquement actif représente un haptène, un antigène, un anticorps ou une protéine.

10. Conjugué oxazinique selon l'une des revendications 6 à 8, caractérisé en ce que le groupe biologiquement actif représente un mono- ou polynucléotide.

11. Procédé de détermination d'une première substance apte à une liaison immunologique, caractérisé en ce qu'on utilise un conjugué selon la revendication 9 avec une deuxième substance apte à une liaison immunologique, qui peut être identique à ou différente de la première substance et en ce qu'on détermine l'absorption ou la variation de fluorescence ou la variation de la polarisation de la fluorescence provoquée par une réaction de liaison immunologique qui est spécifique pour la première substance, à titre de mesure pour la quantité de la substance à déterminer contenue dans l'échantillon.

12. Utilisation d'un conjugué selon la revendication 9 pour des dosages immunologiques.

13. Utilisation d'un conjugué selon la revendication 10 pour l'analyse d'ADN.

14. Procédé de préparation de dérivés oxaziniques répondant à la Formule I selon la revendication 1, caractérisé en ce qu'on condense des 1,3-aminophénols répondant à la Formule III avec des nitrosoaminophénols répondant à la Formule IV.
